# EUROPÄISCHE PATENTSCHRIFT

(11) **EP 3 890 804 B1**
(45) Veröffentlichungstag und Bekanntmachung des Hinweises auf die Patenterteilung: **03.12.2025**
(21) Anmeldenummer: 19794534.8
(22) Anmeldetag: 24.10.2019
(51) Int. Cl.: A61M 5/152, A61M 5/168, G05D 7/00

(54) **INFUSIONSANORDNUNG ZUR VERABREICHUNG EINES MEDIZINISCHEN FLUIDS**
INFUSION ARRANGEMENT FOR ADMINISTRATION OF A MEDICAL FLUID
SYSTÈME DE PERFUSION POUR L'ADMINISTRATION D'UN FLUIDE MÉDICAL

(30) Priorität: 04.12.2018 DE 102018220890
(43) Veröffentlichungstag der Anmeldung: 13.10.2021
(73) Patentinhaber: B. Braun Melsungen AG, 34212 Melsungen (DE)
(72) Erfinder: STETTNER, Jens, 34212 Melsungen (DE); WILDHAGEN, Jens, 30659 Hannover (DE)
(74) Vertreter: Patentanwälte Ruff, Wilhelm, Beier, Dauster & Partner mbB
(86) Internationale Anmeldenummer: PCT/EP2019/079107
(87) Internationale Veröffentlichungsnummer: WO 2020/114677

(56) Entgegenhaltungen:
- EP-A1- 1 321 156
- CA-A- 1 243 251
- CN-A- 101 025 236
- DE-A1- 2 337 947
- US-A- 3 468 308
- US-A1- 2004 171 987

## Beschreibung

Die Erfindung betrifft eine Infusionsanordnung zur Verabreichung eines medizinischen Fluids, aufweisend eine medizinische Elastomerpumpe mit einer elastomeren Membran, die ein Pumpvolumen zur Aufnahme und Förderung des medizinischen Fluids ausbildet, wobei die elastomere Membran in einem wenigstens teilweise mit dem medizinischen Fluid befüllten Füllzustand des Pumpvolumens eine füllzustandsabhängige elastische Dehnung aufweist, und wobei die elastische Dehnung einen dehnungsabhängigen und damit über einer Infusionsdauer veränderlichen Förderdruck auf das Pumpvolumen bewirkt, einen zur Ableitung des medizinischen Fluids aus dem Pumpvolumen vorgesehenen Fluidleitungspfad, der einends fluidleitend mit einem Auslass des Pumpvolumens verbunden ist und andernends fluidleitend mit einem Patientenzugang verbindbar ist, und eine in dem Fluidleitungspfad angeordnete Fluidsteuereinrichtung, die zur Beeinflussung eines mittels der Elastomerpumpe durch den Fluidleitungspfad geförderten Volumenstroms des medizinischen Fluids eingerichtet ist.

Eine derartige Infusionsanordnung ist im Bereich der Medizintechnik allgemein bekannt und zur Verabreichung eines medizinischen Fluids im Rahmen einer Infusionstherapie vorgesehen. Die bekannte Infusionsanordnung weist eine medizinische Elastomerpumpe mit einer elastomeren Membran auf. Solche medizinischen Elastomerpumpen sind im Bereich der Medizintechnik grundsätzlich bekannt und können auch als elastomerische Infusionspumpen bezeichnet werden. Dabei bildet die elastomere Membran ein Pumpvolumen aus, das zur Aufnahme und Förderung des medizinischen Fluids dient. Je nach Füllzustand des Pumpvolumens ist die elastomere Membran mehr oder weniger ballonartig gedehnt und bewirkt einen füllzustandsabhängigen - und damit über einer Infusionsdauer veränderlichen - Förderdruck auf das Pumpvolumen. Zur Ableitung des medizinischen Fluids aus dem Pumpvolumen weist die bekannte Infusionsanordnung einen Fluidleitungspfad auf. Dieser ist einends mit einem Auslass des Pumpvolumens fluidleitend verbunden. Andernends ist der Fluidleitungspfad mit einem Patientenzugang fluidleitend verbindbar. Weiter weist die bekannte Infusionsanordnung eine Fluidsteuereinrichtung auf, die fluidführend in dem Fluidleitungspfad angeordnet ist. Die Fluidsteuereinrichtung dient einer Beeinflussung des mittels der Elastomerpumpe durch den Fluidleitungspfad geförderten Volumenstroms des medizinischen Fluids. Bei der bekannten Infusionsanordnung ist die Fluidsteuereinrichtung in Form eines Flussratenbegrenzers ausgebildet, der auch als Flussdrossel bezeichnet werden kann, und zur Begrenzung des Volumenstroms auf einen vorbestimmten Nominalwert eingerichtet ist. Weiterhin ist aus der US 2004/171987 A1 eine Infusionsanordnung mit einer Fluidsteuereinrichtung in Form eines hydraulischen Stromregelventils bekannt.

Aufgabe der Erfindung ist es, eine Infusionsanordnung der eingangs genannten Art zu schaffen, die eine verbesserte Verabreichung des medizinischen Fluids ermöglicht.

Diese Aufgabe wird dadurch gelöst, dass die Fluidsteuereinrichtung ein hydraulisches Stromregelventil aufweist, das zur Regelung des Volumenstroms auf einen Sollwert eingerichtet ist und mit einer wenigstens in Abhängigkeit des Förderdrucks selbsttätig veränderlichen Drosselwirkung versehen ist. Die Erfindung geht von der Überlegung aus, dass der Förderdruck der Elastomerpumpe bauartbedingt mit abnehmendem Füllzustand des Pumpvolumens sinkt. Zudem wird der Förderdruck im praktischen Gebrauch der Infusionsanordnung durch weitere äußere Faktoren beeinflusst. Solche Faktoren sind insbesondere eine Temperatur des zu verabreichenden medizinische Fluids, eine Umgebungstemperatur, ein externer Druck auf das Pumpvolumen, ein Höhenunterschied zwischen dem Pumpvolumen und dem Patientenzugang, die Art eines patientenseitigen Zugangs (zentral oder peripher) oder dergleichen. Durch die erfindungsgemäße Lösung kann einem Einfluss des aus vorgenannten Gründen bauartbedingt und/oder aufgrund äußerer Faktoren veränderlichen Förderdrucks auf den Volumenstrom entgegengewirkt und eine geregelte Abgabe des medizinischen Fluids ermöglicht werden. Insbesondere kann durch die erfindungsgemäße Regelung des Volumenstroms auf den Sollwert eine - wenigstens im zeitlichen Mittel - verstetigte Abgabe des medizinischen Fluids erreicht werden. Demnach wird eine unbeabsichtigte Unter- oder Überdosierung des medizinischen Fluids vermieden und somit eine verbesserte Verabreichung des medizinischen Fluids ermöglicht. Zu diesem Zweck weist die Fluidsteuereinrichtung das hydraulische Stromregelventil auf. Das Stromregelventil ist zur Regelung des Volumenstroms auf einen Sollwert eingerichtet und ist hierfür mit einer wenigstens in Abhängigkeit des Förderdrucks selbsttätig veränderlichen Drosselwirkung versehen. Bei einem abnehmenden Förderdruck kann die Drosselwirkung selbsttätig verringert und damit der Volumenstrom in Richtung des Sollwerts erhöht werden. Bei einem beispielsweise durch genannte äußere Faktoren erhöhten Förderdruck kann die Drosselwirkung selbsttätig erhöht und damit der Volumenstrom in Richtung des Sollwerts verringert werden. Der grundsätzliche Aufbau und die grundsätzliche Funktionsweise hydraulischer Stromregelventile sind im Bereich der Fluidtechnik als solche allgemein bekannt. Insoweit kann das Stromregelventil insbesondere als 2- oder 3-Wege-Stromregelventil, Differenzdruck-Volumenstromregler oder dergleichen ausgebildet sein. Die medizinische Elastomerpumpe ist vorzugsweise derart gestaltet, dass auf eine externe Energieversorgung zur Generierung des Förderdrucks und/oder sonstiger Funktionen der medizinischen Elastomerpumpe verzichtet werden kann. Dementsprechend ist das Stromregelventil vorzugsweise derart gestaltet, dass auf eine externe Energieversorgung zur Regelung des Volumenstroms verzichtet werden kann. Der Fluidleitungspfad kann insbesondere durch einen Fluidleitungskanal, eine Schlauchleitung, eine Rohrleitung oder dergleichen ausgebildet sein. Die Fluidsteuereinrichtung bildet dabei einen Abschnitt des Fluidleitungspfads und ist im Betrieb der medizinischen Elastomerpumpe im Volumenstrom des medizinischen Fluids angeordnet. Die Fluidsteuereinrichtung kann separat von der medizinischen Elastomerpumpe ausgebildet und/oder angeordnet und insbesondere zwischen zwei dem Pumpvolumen nachgeordneten Abschnitten des Fluidleitungspfads fluidführend angeordnet sein. Alternativ kann die Fluidsteuereinrichtung baulich in die Elastomerpumpe integriert sein.

In Ausgestaltung der Erfindung weist das Stromregelventil eine stetige Regelcharakteristik auf, wobei die Drosselwirkung selbsttätig stetig veränderlich ist, oder das Stromregelventil weist eine unstetige 2-Punkt-Regelcharakteristik auf, wobei die Drosselwirkung selbsttätig wechselweise zwischen einer Sperrung und einer Freigabe des Volumenstroms veränderlich ist. Regler mit stetiger und Regler mit unstetiger 2-Punkt-Regelcharakteristik sind im Bereich der Regelungstechnik als solche grundsätzlich bekannt. Dabei hat sich die erstgenannte Variante mit stetiger Regelcharakteristik insbesondere für Infusionsanordnungen zur Verabreichung einer kontinuierlichen Basalrate des medizinischen Fluids als vorteilhaft erwiesen. Die zweitgenannte Variante mit unstetiger Regelcharakteristik ist insbesondere für Infusionsanordnungen vorteilhaft, die einer Bolusverabreichung des medizinischen Fluids dienen. Hierbei wechselt die Drosselwirkung des Stromregelventils selbsttätig zwischen einem abgesperrten Sperrzustand und einem freigegebenen Freigabezustand. Im Sperrzustand des Stromregelventils findet kein Volumenstrom statt. Demgegenüber ist im Freigabezustand der Volumenstrom freigegeben. Weiter hat sich gezeigt, dass die unstetige 2-Punkt-Regelcharakteristik einer unerwünschten Verstopfung der Infusionsanordnung entgegenwirken kann. Denn durch das selbsttätige wechselweise Verändern der Drosselwirkung zwischen Sperrung und Freigabe des Volumenstroms wird einem unerwünschten Anhaften von Partikeln im Fluidleitungspfad und damit einer Verstopfung desselben entgegenwirkt.

Weiter gemäß der Erfindung ist das hydraulische Stromregelventil ein 2-Wege-Stromregelventil und weist einen ersten Strömungswiderstand auf, an dem einlassseitig der Förderdruck und auslassseitig ein Auslassdruck anliegt, und weist einen zweiten Strömungswiderstand auf, der die selbsttätig veränderliche Drosselwirkung aufweist und dem ersten Strömungswiderstand in Förderrichtung des Volumenstroms nachgelagert ist, und weist eine Druckwaagenanordnung mit einem hydraulisch verlagerbaren Stellelement auf, das einends mit dem Förderdruck und andernends mit dem Auslassdruck beaufschlagt ist und mittels dessen der zweite Strömungswiderstand in Abhängigkeit einer differenzdruckbedingten Verlagerung des Stellelements selbsttätig veränderlich ist. 2-Wege-Stromregelventile sind im Bereich der Fluidtechnik als solches grundsätzlich bekannt. Vorliegend weist das 2-Wege-Stromregelventil einen ersten Strömungswiderstand auf, der auch als Messdrossel oder Messblende bezeichnet werden kann. An dem ersten Strömungswiderstand liegt ein Druckabfall an, der sich aus dem einlassseitig anliegenden Förderdruck und dem auslassseitigen Auslassdruck ergibt. Der Druckabfall kann auch als Differenzdruck oder Druckdifferenz bezeichnet werden. Der Auslassdruck ist dabei der hydraulische Druck, der an dem patientenseitigen Ende des Fluidleitungspfads anliegt. Der zweite Strömungswiderstand ist stromabwärts des ersten Strömungswiderstands angeordnet und mittels des Stellelements der Druckwaagenanordnung im Hinblick auf seine Drosselwirkung selbsttätig veränderlich. Dabei ist das Stellelement der Druckwaagenanordnung einends mit dem Förderdruck und andernends mit dem Auslassdruck beaufschlagt und differenzdruckbedingt verlagerbar zur Veränderung der Drosselwirkung des zweiten Strömungswiderstands. Die Ausgestaltung des hydraulischen Stromregelventils als 2-Wege-Stromregelventil ist insbesondere im Hinblick auf eine Zuverlässigkeit und Robustheit der Regelung des Volumenstroms besonders vorteilhaft.

In weiterer Ausgestaltung der Erfindung ist der erste Strömungswiderstand ein den Fluidleitungspfad lokal verengendes Kapillarelement, das eine im Vergleich zu dem Förderdruck geringe Druckdifferenz zwischen Förderdruck und Auslassdruck bewirkt, wobei vorzugsweise die Druckdifferenz um einen Faktor 10 bis 20, bevorzugt 20 bis 100, besonders bevorzugt 100 bis 1.000, geringer ist als der Förderdruck. Demnach weist der erste Strömungswiderstand einen vergleichsweise geringen hydraulischen Widerstand auf. Hierdurch kann insbesondere einer ungewollten Verstopfung des Fluidleitungspfads entgegengewirkt werden. Das Kapillarelement kann insbesondere als Röhrchen, lokal verengter Schlauchabschnitt oder Rohrabschnitt ausgebildet sein. Vorzugsweise ist der hydraulische Widerstand des Kapillarelements nicht einstellbar.

In weiterer Ausgestaltung der Erfindung weist das Kapillarelement einen strömungswirksamen Querschnitt auf, der wenigstens 100 µm, bevorzugt 100 µm bis 150 µm, beträgt. Durch diese Ausgestaltung der Erfindung kann auf besonders einfache Weise einer Verstopfung des Fluidleitungspfads im Bereich des Kapillarelements entgegengewirkt werden. Dabei hat sich gezeigt, dass ein wirksamer Querschnitt von 100 µm bis 150 µm bei Verwendung von medizinischen Fluiden, wie sie insbesondere bei einer Chemo- oder Antibiotikatherapie Verwendung finden, besonders vorteilhaft ist.

In weiterer Ausgestaltung der Erfindung weist die Druckwaagenanordnung ein mit dem Stellelement wirkverbundenes Federelement auf, das eine Federkraft auf das Stellelement bewirkt. Das Federelement dient einer federkraftbedingten Rückstellung oder Vorspannung des Stellelements. Durch das Federelement kann eine verbesserte Gleichgewichtslage des Stellelements im Regelbetrieb und damit ein verbessertes Betriebsverhalten des 2-Wege-Stromregelventils erreicht werden.

In weiterer Ausgestaltung der Erfindung ist eine Verstelleinrichtung vorgesehen, mittels derer die Federkraft des Federelements manuell einstellbar ist. Die Verstelleinrichtung dient somit mittelbar einer Einstellung einer Gleichgewichtslage des Stellelements und damit einer Einstellung einer Soll-Druckdifferenz zwischen Förderdruck und Auslassdruck. Diese Ausgestaltung der Erfindung ist besonders dann vorteilhaft, wenn der erste Strömungswiderstand als nicht einstellbarer Strömungswiderstand ausgebildet ist. In diesem Fall kann mittels der Verstelleinrichtung eine Einstellung der Soll-Druckdifferenz und damit des Sollwerts des Volumenstroms erreicht werden. Dies, da der Volumenstrom gemäß der hier einschlägigen physikalischen Zusammenhänge proportional zur Druckdifferenz ist.

In weiterer Ausgestaltung der Erfindung ist das Stellelement in Form eines Druckkolbens oder in Form einer Druckmembran ausgebildet. Durch die Ausbildung des Stellelements in Form eines Druckkolbens kann eine besonders robuste und einfach zu fertigende Ausgestaltung des 2-Wege-Stromregelventils erreicht werden. Die zweite Variante, bei der das Stellelement in Form einer Druckmembran ausgebildet ist, ist besonders unempfindlich gegenüber Verunreinigung und einem etwaigen durch das geförderte medizinische Fluid bedingten Verkleben oder Festsetzen des Stellelements.

In weiterer Ausgestaltung der Erfindung weist die Druckmembran eine randseitig umlaufende Sickenanordnung nach Art einer Lautsprechermembran auf. Die Sickenanordnung bewirkt, dass die Druckmembran selbst nur eine sehr geringe Rückstellkraft gegenüber einer differenzdruckbedingten Verlagerung aufweist. Dadurch ist die Druckmembran auch bei sehr geringen Druckdifferenzen zwischen Förderdruck und Auslassdruck zuverlässig verlagerbar.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer, teilweise aufgeschnittener Darstellung eine Ausführungsform einer erfindungsgemäßen Infusionsanordnung mit einer zeichnerisch abstrahiert dargestellten Fluidsteuereinrichtung,
- Fig. 2: die Infusionsanordnung nach Fig. 1 in einer stark vereinfachten schematischen Darstellung nach Art eines hydraulischen Blockschaltbildes,
- Fig. 3: die Infusionsanordnung nach den Fig. 1 und 2 in einer weiteren schematischen Darstellung, die Einzelheiten der Fluidsteuereinrichtung zeigt,
- Fig. 4: in einer schematischen Darstellung entsprechend Fig. 3 eine weitere Ausführungsform einer erfindungsgemäßen Infusionsanordnung und
- Fig. 5: eine schematische Detaildarstellung einer Druckmembran der Infusionsanordnung nach den Fig. 1 bis 3.

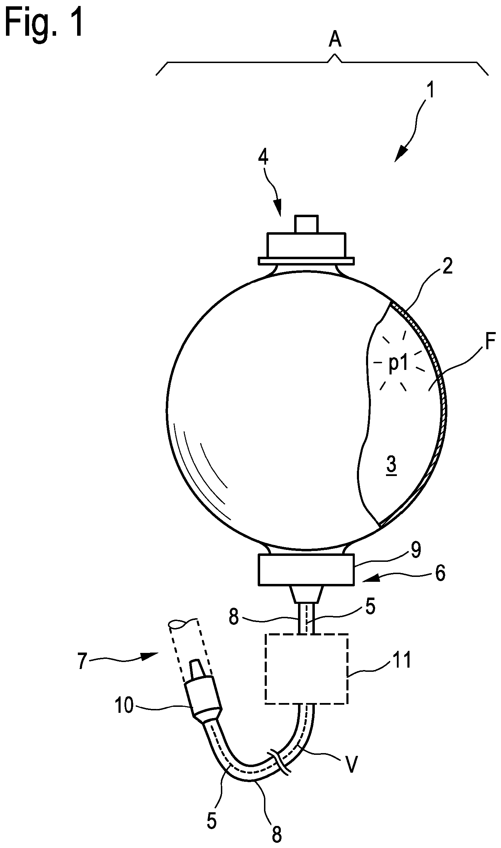

Gemäß Fig. 1 ist eine Infusionsanordnung A zur Verabreichung eines medizinischen Fluids F im Rahmen einer ambulanten und/oder stationären Infusionstherapie vorgesehen. Die Infusionsanordnung A weist eine medizinische Elastomerpumpe 1 auf, die auch als elastomerische Infusionspumpe bezeichnet werden kann. Die medizinische Elastomerpumpe 1 weist eine elastomere Membran 2 auf, die ein Pumpvolumen 3 zur Aufnahme und Förderung des medizinischen Fluids F ausbildet. In der anhand Fig. 1 ersichtlichen Konfiguration ist das Pumpvolumen 3 in einem mit dem medizinischen Fluid F befüllten Füllzustand gezeigt. In diesem Füllzustand weist die elastomere Membran 2 eine füllzustandsabhängige elastische Dehnung auf und ist ballonartig weichelastisch gedehnt. Die elastische Dehnung der Membran 2 bewirkt einen dehnungsabhängigen - und damit implizit füllzustandsabhängigen - veränderlichen Förderdruck p1 auf das Pumpvolumen 3. Die Membran 2 ist anhand Fig. 1 aus zeichnerischen Gründen mit einer überhöhten Wandungsstärke dargestellt. Zum Befüllen des Pumpvolumens 3 mit dem medizinischen Fluid F weist die Elastomerpumpe 1 einen wiederverschließbaren Einfüllstutzen 4 auf, der auf grundsätzlich bekannte Weise fluiddicht an die Membran 2 angeschlossen ist.

Zur Ableitung des medizinischen Fluids F aus dem Pumpvolumen 3 weist die Infusionsanordnung A einen Fluidleitungspfad 5 auf, der einends fluidleitend mit einem Auslass 6 des Pumpvolumens 3 verbunden ist und der andernends fluidleitend mit einem Patientenzugang 7 verbindbar ist. Der Fluidleitungspfad 5 ist vorliegend mittels einer flexiblen Schlauchleitung 8 ausgebildet, die einstückig oder in mehreren fluidleitend miteinander verbundenen Teilabschnitten angeordnet sein kann. An ihrem dem Auslass 6 zugewandten Stirnende ist die Schlauchleitung 8 auf grundsätzlich bekannte Weise fest und fluiddicht mit einem dem Auslass 6 zugeordneten Auslassstutzen 9 der Elastomerpumpe 1 verbunden. Der Auslassstutzen 9 bildet eine fluidführende Überleitung von dem Pumpvolumen 3 über den Auslass 6 in den Fluidleitungspfad 5. An ihrem dem Patientenzugang 7 zugewandten Stirnende weist die Schlauchleitung 8 ein Anschlusselement 10 auf, das vorliegend exemplarisch in Form eines schematisch stark vereinfachten Luer-Anschlusses ausgebildet ist. Bei einer nicht dargestellten Ausführungsform kann das Anschlusselement 10 in Form eines NRFif-Anschlusses ausgebildet sein. Der Luer-Anschluss 10 ist zur fluidleitenden Verbindung mit dem Patientenzugang 7 vorgesehen. Der Patientenzugang 7 ist anhand Fig. 1 lediglich stark vereinfacht schematisch und teilweise abgeschnitten dargestellt.

Die Infusionsanordnung A weist zudem eine in dem Fluidleitungspfad 5 angeordnete Fluidsteuereinrichtung 11 auf. Die Fluidsteuereinrichtung 11 ist zur Beeinflussung eines mittels der Elastomerpumpe 1 durch den Fluidleitungspfad 5 geförderten Volumenstroms V des medizinischen Fluids F eingerichtet. Die Fluidsteuereinrichtung 11 ist fluidführend und insoweit einends an ein - in Bezug auf die Zeichenebene der Fig. 1 - oberes nicht näher bezeichnetes Segment der Schlauchleitung 8 und andernends an ein unteres nicht näher bezeichnetes Segment der Schlauchleitung 8 fluidleitend angeschlossen. Der Fluidleitungspfad 5 erstreckt sich somit wenigstens abschnittsweise durch die Fluidsteuereinrichtung 11.

Die Infusionsanordnung A ist vorliegend derart dimensioniert, dass sie ohne weiteres von einem Patienten am Körper getragen werden kann. Dabei sind sowohl die Elastomerpumpe 1 als auch die Fluidsteuereinrichtung 11 derart gestaltet, dass diese jeweils ohne eine externe Energieversorgung betreibbar sind. Demnach sind keinerlei elektrische Versorgungsleitungen oder elektrische Energiespeichereinheiten zur Versorgung der Elastomerpumpe 1 und/oder der Fluidsteuereinrichtung 11 mit elektrischer Betriebsenergie notwendig. Die Elastomerpumpe 1 ist dementsprechend leicht und kompakt dimensioniert, wobei das Pumpvolumen 3 vorliegend nominal mit 400 ml bemessen ist. Es versteht sich, dass das Pumpvolumen 3 auch hiervon abweichend, beispielsweise mit 50 ml bis 1.000 ml, bemessen sein kann.

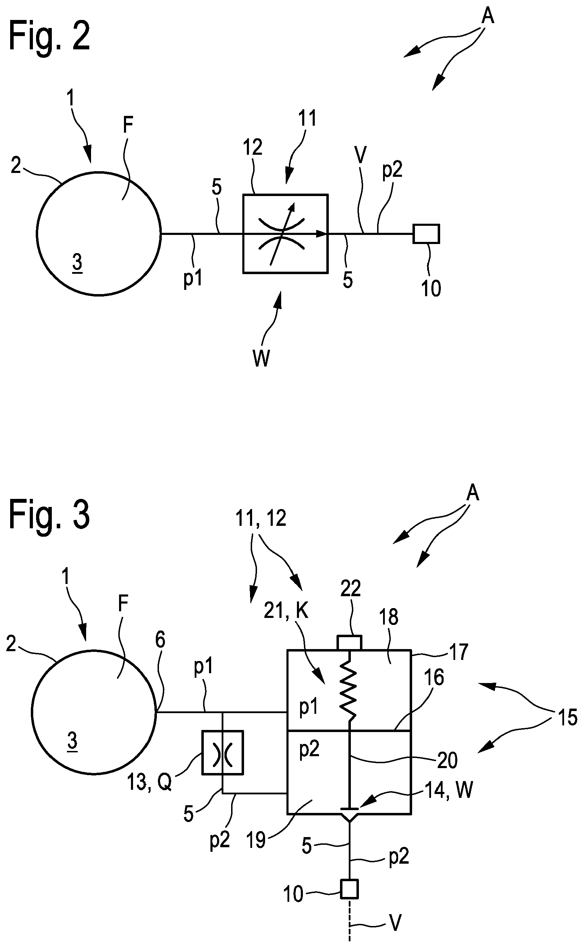

Wie weiter anhand Fig. 2 ersichtlich ist, weist die Fluidsteuereinrichtung 11 ein hydraulisches Stromregelventil 12 auf. Das Stromregelventil 12 ist zur Regelung des Volumenstroms V auf einen nicht näher bezeichneten Sollwert eingerichtet und mit einer wenigstens in Abhängigkeit des Förderdrucks p1 selbsttätig veränderlichen Drosselwirkung W versehen. Die selbsttätig veränderliche Drosselwirkung W bewirkt somit einen mittels des Stromregelventils 12 selbsttätig veränderlichen Druckabfall Δp zwischen einem Einlass und einem Auslass der Fluidsteuereinrichtung 11. Dieser Druckabfall Δp kann auch als Druckdifferenz Δp oder Differenzdruck Δp bezeichnet werden und setzt sich aus dem einlassseitig anliegenden Förderdruck p1 und einem auslassseitig der Fluidsteuereinrichtung 11 und damit an dem Anschlusselement 10 anliegenden Auslassdruck p2 zusammen. Unter der vereinfachenden Annahme einer rein laminaren stationären Strömung und eines homogenen Newton'schen Fluids lässt sich der Volumenstrom V im vorliegenden Fall aufgrund der Gesetzmäßigkeit von Hagen-Poiseuille beschreiben. Demgemäß ist der Volumenstrom V proportional zum Differenzdruck Δp.

Über der Dauer der Infusionstherapie, die auch als Infusionsdauer bezeichnet werden kann, nimmt der Füllzustand des Pumpvolumens 3 aufgrund einer Ableitung des medizinischen Fluids F aus demselben ab. Durch diese Abnahme des Füllzustands verringert sich die elastische Dehnung der Membran 2 und damit naturgemäß auch der dehnungsabhängige Förderdruck p1. Ohne einen regelnden Eingriff würde diese füllzustandsbedingte Abnahme des Förderdrucks p1 zwangsläufig zu einer unerwünschten Abnahme des Volumenstroms V führen. Um dies auszugleichen, ist die Drosselwirkung W auf noch näher zu beschreibende Weise in Abhängigkeit des Förderdrucks p1 veränderlich. Genauer ist die Drosselwirkung W bei einer füllzustandsbedingten Abnahme des Förderdrucks p1 selbsttätig verringerbar, so dass der Differenzdruck Δp zwischen Förderdruck p1 und Auslassdruck p2 auf einen Druck-Sollwert geregelt ist. Damit ist auch der Volumenstrom V auf den besagten Sollwert regelbar. Gleichermaßen kann bei einem aufgrund äußerer Faktoren erhöhten Förderdruck p1 eine dementsprechende Erhöhung der Drosselwirkung W erfolgen. Weitere Details des Stromregelventils 12 sind insbesondere anhand Fig. 3 ersichtlich.

Wie anhand Fig. 3 ersichtlich ist, weist das hydraulische Stromregelventil 12 einen ersten Strömungswiderstand 13, einen zweiten Strömungswiderstand 14 und eine Druckwaagenanordnung 15 mit einem hydraulisch verlagerbaren Stellelement 16 auf. Das hydraulische Stromregelventil ist insoweit in Form eines 2-Wege-Stromregelventils 12 ausgebildet.

Der erste Strömungswiderstand 13 ist vorliegend in Form eines Kapillarelements ausgebildet. Das Kapillarelement 13 bildet eine lokale Verengung des Fluidleitungspfads 5 aus und ist einlassseitig fluidleitend mit dem Auslass 6 der Elastomerpumpe 1 fluidleitend verbunden. Einlassseitig liegt somit der Förderdruck p1 an dem Kapillarelement 13 an.

Die Druckwaagenanordnung 15 ist schematisch stark vereinfacht dargestellt und in Form einer Membran-Druckwaage ausgebildet. Das Stellelement ist insoweit in Form einer Druckmembran 16 ausgebildet. Die Druckmembran 16 ist auf einer - in Bezug auf die Zeichenebene der Fig. 3 - oberen Membranseite mit dem Förderdruck p1 beaufschlagt und auf einer unteren Membranseite mit dem Auslassdruck p2 druckbeaufschlagt. Dabei ist die Druckmembran 16 randseitig fluiddicht auf grundsätzlich bekannte Weise in ein Druckgehäuse 17 eingepasst. Das Druckgehäuse 17 weist eine obere Druckkammer 18, die der Oberseite der Membran 16 zugeordnet ist, und eine untere Druckkammer 19 auf, die der Unterseite der Druckmembran 16 zugeordnet ist. Die Druckmembran 16 ist auf grundsätzlich bekannte Weise differenzdruckbedingt in Hochrichtung des Druckgehäuses 17 verlagerbar und unterseitig mit einem Stößelelement 20 versehen, das gemeinsam mit einer gehäuseseitigen nicht näher bezeichneten Auslassöffnung den zweiten Strömungswiderstand 14 ausbildet. Der zweite Strömungswiderstand 14 weist die selbsttätig veränderliche Drosselwirkung W auf, die im vorliegenden Fall mittels einer Verlagerung der Druckmembran 16 differenzdruckbedingt veränderlich ist. Stromabwärts des zweiten Strömungswiderstands 14 mündet der Fluidleitungspfad 5 in das Anschlusselement 10.

Zudem ist ein Federelement 21 vorgesehen, das vorliegend in der oberen Druckkammer 18 angeordnet ist, und eine Federkraft K auf die Druckmembran 16 bewirkt. Die Federkraft K wirkt einer Verlagerung der Druckmembran 16 in Richtung der oberen Druckkammer 18 entgegen. Das Federelement 21 ist vorliegend in Form einer Spiralfeder ausgebildet, die einends an dem Druckgehäuse 17 und andernends oberseitig an der Druckmembran 16 abgestützt ist.

Zur Erläuterung der Funktion der Fluidsteuervorrichtung 11 wird nachfolgend zunächst von einem stationären Zustand ausgegangen, in dem die obere Druckkammer 18 mit dem Förderdruck p1 und die untere Druckkammer 19 mit dem Auslassdruck p2 beaufschlagt ist. Insoweit stellt sich über dem Kapillarelement 13 der Druckabfall Δp ein, wobei der sich durch das Auslasselement 10 ergießende Volumenstrom V proportional zu dem Druckabfall bzw. Differenzdruck Δp ist. In diesem Zustand befindet sich die Druckwaagenanordnung 15 in einem Kräftegleichgewicht, in dem oberseitig der Druckmembran die Federkraft K und der Förderdruck p1 an der Druckmembran 16 angreifen und unterseitig allein der Auslassdruck p2 angreift. Dabei ist die Druckmembran 16 vorliegend ober- und unterseitig gleich dimensioniert, so dass jeweils eine gleich bemessene druckbeaufschlagte Fläche vorliegt. In diesem Zustand, der auch als Regelgleichgewicht bezeichnet werden kann, folgt aus einer Kräftegleichgewichtsbetrachtung an der Druckmembran 16, dass der Differenzdruck Δp gleich dem Quotienten aus Federkraft K und druckbeaufschlagter Fläche der Druckmembran 16 ist. Somit ist der Differenzdruck Δp und damit der Volumenstrom V aufgrund der vorbeschriebenen Gesetzmäßigkeit unabhängig vom Förderdruck p1 und unabhängig von dem Auslassdruck p2.

Sinkt nun der Förderdruck p1 füllzustandsbedingt ab, bewegt sich die Druckmembran 16 ausgehend von dem vorbeschriebenen Gleichgewichtszustand entgegen der Federkraft K nach oben, wodurch der zweite Strömungswiderstand 14 über das Stößelelement 20 beeinflusst wird. Genauer wird der zweite Strömungswiderstand 14 hierbei im Hinblick auf seine Drosselwirkung W verringert. Dies geschieht vorliegend auf grundsätzlich bekannte Weise durch eine Vergrößerung eines wirksamen Strömungsquerschnitts des zweiten Strömungswiderstands 14. Hierdurch sinkt der Auslassdruck p2 dementsprechend, wobei der Differenzdruck Δp gemäß dem vorbeschriebenen Kräftegleichgewicht konstant geregelt wird. Hierdurch wird wiederum der Volumenstrom V auf den zum Differenzdruck Δp proportionalen Sollwert geregelt. Entsprechendes gilt in umgekehrter Weise bei einer unerwünschten zeitweisen Erhöhung des Förderdrucks p1 infolge äußerer Faktoren, wie beispielsweise einer Temperaturerhöhung oder einer äußeren Druckbelastung der elastomeren Membran 2.

Das Kapillarelement 13 weist vorliegend einen nicht einstellbaren Strömungswiderstand auf. Um dennoch eine Einstellbarkeit des Volumenstroms V zu ermöglichen, ist vorliegend eine Verstelleinrichtung 22 vorgesehen, mittels derer die Federkraft K des Federelements 21 manuell einstellbar ist. Die Verstelleinrichtung 22 ist oberseitig des Druckgehäuses 17 angeordnet und derart gestaltet, dass eine verstärkte und/oder verringerte axiale Vorspannung des Federelements 21 einstellbar ist. Zu diesem Zweck kann die Verstelleinrichtung 21 beispielsweise mit einem Gewinde in das Druckgehäuse 17 eingeschraubt sein, das eine rotatorische manuelle Verstellbewegung der Verstelleinrichtung 22 in eine axiale Vorspannbewegung zur Beeinflussung der Federkraft K umsetzt. Wie oben dargelegt, ist der Differenzdruck Δp und damit der Volumenstrom V im Regelgleichgewicht, d.h. bei einem Kräftegleichgewicht der Druckmembran 16, proportional zum Quotienten aus Federkraft K und Druckfläche der Druckmembran 16. Demnach kann durch eine manuelle Verstellung der Federkraft K mittels der Verstelleinrichtung 22 auf einfache und wirksame Weise der Volumenstrom V im Gleichgewichtszustand eingestellt werden. Der Volumenstrom V im Gleichgewichtszustand des Stromregelventils 11 gibt den Sollwert vor, auf den der Volumenstrom V zu regeln ist.

Vorliegend bewirkt das Kapillarelement 13 einen im Vergleich zu dem Förderdruck p1 geringen Druckabfall Δp. Dementsprechend ist ein strömungswirksamer Querschnitt Q des Kapillarelements 13 vergleichsweise groß bemessen. Im vorliegenden Fall beträgt der strömungswirksame Querschnitt Q des Kapillarelements 13 in etwa 150 µm.

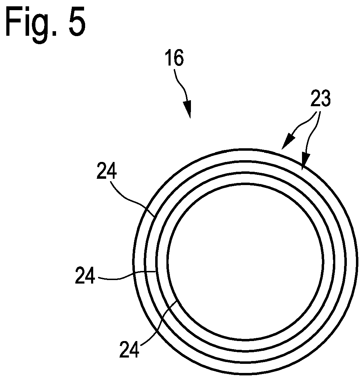

Weitere Einzelheiten der Druckmembran 16 sind anhand Fig. 5 schematisch verdeutlicht. Dort ist erkennbar, dass die Druckmembran 16 eine randseitig umlaufende Sickenanordnung 23 aufweist. Die Sickenanordnung 23 ist nach Art einer Lautsprechermembran ausgebildet und weist mehrere konzentrische Sicken 24 auf. Vorliegend sind drei Sicken 24 vorgesehen. Die Sickenanordnung 23 bewirkt, dass die Druckmembran 16 wenigstens im Wesentlichen, vorzugsweise nahezu vollständig, frei von Rückstellkräften ist. Dies ermöglicht insbesondere eine verbesserte Einstellbarkeit des Regelgleichgewichts des Stromregelventils 12.

Wie anhand der vorstehenden Funktionsbeschreibung des Stromregelventils 12 verdeutlich ist, weist das Stromregelventil 12 in der anhand Fig. 3 dargestellten Ausgestaltung eine stetige Regelcharakteristik auf. Dies bedeutet, dass die Drosselwirkung W selbsttätig stetig - d.h. zwischen einer Vielzahl unterschiedlicher Werte kontinuierlich - veränderlich ist.

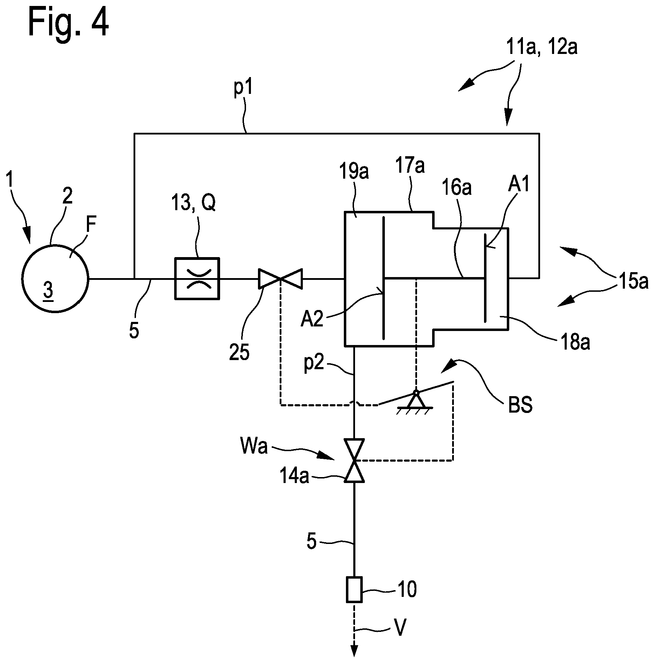

Demgegenüber weist das anhand Fig. 4 ersichtliche Stromregelventil 12a eine unstetige 2-Punkt-Regelcharakteristik auf, wobei die Drosselwirkung Wa selbsttätig wechselweise zwischen einer Sperrung und einer Freigabe des Volumenstroms V veränderlich ist. Nachfolgend wird lediglich auf die wesentlichen Unterschiede der anhand Fig. 4 ersichtlichen Ausführungsform gegenüber der Ausführungsform nach Fig. 3 eingegangen. Baugleiche Elemente und Abschnitte sind dabei mit identischen Bezugszeichen versehen. Merkmale die gegenüber der Ausführungsform nach Fig. 3 eine unterschiedliche konstruktive und/oder funktionelle Ausgestaltung aufweisen, sind unter Hinzufügung des Kleinbuchstabens a gekennzeichnet. Im Übrigen wird zur Vermeidung von Wiederholungen auf die in Zusammenhang mit den Fig. 1 bis 3 stehende Offenbarung verwiesen, die in entsprechender Weise für die Ausführungsform nach Fig. 4 gilt.

Die Ausführungsform nach Fig. 4 unterscheidet sich im Wesentlichen durch die unterschiedliche Ausgestaltung der Druckwaagenanordnung 15a und die damit einhergehende unstetige 2-Punkt-Regelcharakteristik von der Ausführungsform nach Fig. 3. Die Druckwaagenanordnung 15a weist ein hydraulisch verlagerbares Stellelement in Form eines Druckkolbens 16a auf. Der Druckkolben 16a ist in einem Druckgehäuse 17a hydraulisch verlagerbar geführt und weist vorliegend unterschiedliche druckbeaufschlagbare Kolbenflächen auf. Eine erste Kolbenfläche A1 ist in einer ersten Druckkammer 18a des Druckgehäuses 17a angeordnet. Eine zweite Kolbenfläche A2 ist in einer zweiten Druckkammer 19a des Druckgehäuses 17a angeordnet. Die erste Kolbenfläche A1 ist mit dem Förderdruck p1 druckbeaufschlagt. Die zweite Kolbenfläche A2 ist mit dem Auslassdruck p2 druckbeaufschlagt.

Weiter im Unterschied zu der Ausführungsform nach Fig. 3 ist der zweite Strömungswiderstand in Form eines Absperrventils 14a ausgebildet. Das Absperrventil 14a ist stromabwärts der zweiten Druckkammer 19a in dem Fluidleitungspfad 5 angeordnet und über einen schematisch angedeuteten bistabilen Kippschalter BS zwischen einer Sperrstellung und einer Freigabestellung verlagerbar. In der Sperrstellung ist der Volumenstrom V mittels des Absperrventils 14a abgesperrt. In der Freigabestellung ist der Volumenstrom V demgegenüber freigegeben. Der bistabile Kippschalter BS ist in Abhängigkeit einer differenzdruckbedingten Verlagerung des Druckkolbens 16a betätigbar, was anhand der nicht näher bezeichneten strichlierten Wirkverbindung zwischen dem bistabilen Kippschalter BS und dem Druckkolben 16a verdeutlicht ist. Der erste Strömungswiderstand 13 entspricht dem ersten Strömungswiderstand 13 der Infusionsanordnung A nach Fig. 3. Zudem ist ein weiteres Absperrventil 25 vorgesehen, das in bestimmungsgemäßer Förderrichtung des medizinischen Fluids F stromabwärts des ersten Strömungswiderstands 13 und stromaufwärts der zweiten Druckkammer 19a angeordnet ist. Das zweite Absperrventil 25 ist ebenfalls mittels des bistabilen Kippschalters BS zwischen einer Sperrstellung und einer Freigabestellung verlagerbar. Dies ist mittels der strichliert angedeuteten Wirkverbindung zwischen dem Kippschalter BS und dem zweiten Absperrventil 25 angedeutet. Der sich einstellende Differenzdruck Δp zwischen Förderdruck p1 und Auslassdruck p2 ist bei dieser Ausgestaltung der Erfindung durch die Größenverhältnisse der ersten Kolbenfläche A1 und der zweiten Kolbenfläche A2 definiert.

Das Absperrventil 14a und das zweite Absperrventil 25 sind mittels der differenzdruckbedingten hydraulischen Verlagerung des Druckkolbens 16a insoweit wechselweise mittels des Kippschalters BS schaltbar, als das erste Absperrventil 14a immer dann gesperrt ist, wenn das zweite Absperrventil 25 freigegeben ist und umgekehrt.

In einem ersten Zustand ist das zweite Absperrventil 25 freigegeben und das erste Absperrventil 14a ist gesperrt. Aufgrund der unterschiedlichen Größenverhältnisse der Kolbenflächen A1, A2 bewegt sich der Druckkolben 16a - in Bezug auf die Zeichenebene der Fig. 4 - nach rechts. Hierbei wird die zweite Druckkammer 19a in verstärktem Umfang befüllt, die erste Druckkammer 18a demgegenüber entleert.

Ab einer vorbestimmten Position des Druckkolbens 16a wird der bistabile Kippschalter BS über die Wirkverbindung zu dem Druckkolben 16a betätigt, wobei das zweite Absperrventil 25 gesperrt und das erste Absperrventil 14a freigegeben wird. In diesem zweiten Zustand bewirkt der auf die Kolbenfläche A1 wirkende Förderdruck p1 eine Bewegung des Druckkolbens 16a nach links, so dass die zweite Druckkammer 19a über den Fluidleitungspfad 5 in Richtung des Anschlusselements 10 entleert wird. Hierbei stellt sich der Volumenstrom V ein. Ab einer gewissen Grenzstellung wird hierbei wiederum der bistabile Kippschalter BS mittels des Druckkolbens 16a betätigt, wodurch der Schaltzustand der Absperrventile 14a, 25 wechselweise geändert wird. Hiernach wird der vorbeschriebene Zyklus erneut durchlaufen. Die vorbeschriebene 2-Punkt-Regelcharakteristik des Stromregelventils 12a ist besonders vorteilhaft im Hinblick auf eine - im zeitlichen Mittel volumenstromgeregelte - bolusartige Abgabe des medizinischen Fluids F.

## Patentansprüche

1. Infusionsanordnung (A) zur Verabreichung eines medizinischen Fluids (F), aufweisend
- eine medizinische Elastomerpumpe (1) mit einer elastomeren Membran (2), die ein Pumpvolumen (3) zur Aufnahme und Förderung des medizinischen Fluids (F) ausbildet, wobei die elastomere Membran (2) in einem wenigstens teilweise mit dem medizinischen Fluid (F) befüllten Füllzustand des Pumpvolumens (3) eine füllzustandsabhängige elastische Dehnung aufweist, und wobei die elastische Dehnung einen dehnungsabhängigen und damit über einer Infusionsdauer veränderlichen Förderdruck (p1) auf das Pumpvolumen (3) bewirkt,
- einen zur Ableitung des medizinischen Fluids (F) aus dem Pumpvolumen (3) vorgesehenen Fluidleitungspfad (5), der einends fluidleitend mit einem Auslass (6) des Pumpvolumens (3) verbunden ist und andernends fluidleitend mit einem Patientenzugang (7) verbindbar ist, und
- eine in dem Fluidleitungspfad (5) angeordnete Fluidsteuereinrichtung (11, 11a), die zur Beeinflussung eines mittels der Elastomerpumpe (1) durch den Fluidleitungspfad (5) geförderten Volumenstroms (V) des medizinischen Fluids (F) eingerichtet ist,
- wobei die Fluidsteuereinrichtung (11, 11a) ein hydraulisches Stromregelventil (12, 12a) aufweist, das zur Regelung des Volumenstroms (V) auf einen Sollwert eingerichtet ist und mit einer wenigstens in Abhängigkeit des Förderdrucks (p1) selbsttätig veränderlichen Drosselwirkung (W, Wa) versehen ist, **dadurch gekennzeichnet,**
**dass** das hydraulische Stromregelventil ein 2-Wege-Stromregelventil (12, 12a) ist und einen ersten Strömungswiderstand (13) aufweist, an dem einlassseitig der Förderdruck (p1) und auslassseitig ein Auslassdruck (p2) anliegt, und einen zweiten Strömungswiderstand (14, 14a) aufweist, der die selbsttätig veränderliche Drosselwirkung (W, Wa) aufweist und dem ersten Strömungswiderstand (13) in Förderrichtung des Volumenstroms (V) nachgelagert ist, und eine Druckwaagenanordnung (15, 15a) mit einem hydraulisch verlagerbaren Stellelement (16, 16a) aufweist, das einends mit dem Förderdruck (p1) und andernends mit dem Auslassdruck (p2) beaufschlagt ist und mittels dessen der zweite Strömungswiderstand (14, 14a) in Abhängigkeit einer differenzdruckbedingten Verlagerung des Stellelements (16, 16a) selbsttätig veränderlich ist.

2. Infusionsanordnung (A) nach Anspruch 1, **dadurch gekennzeichnet, dass** das Stromregelventil (12) eine stetige Regelcharakteristik aufweist, wobei die Drosselwirkung (W) selbsttätig stetig veränderlich ist, oder dass das Stromregelventil (12a) eine unstetige 2-Punkt-Regelcharakteristik aufweist, wobei die Drosselwirkung (Wa) selbsttätig wechselweise zwischen einer Sperrung und einer Freigabe des Volumenstroms (V) veränderlich ist.

3. Infusionsanordnung (A) nach Anspruch 1 oder 2, **dadurch gekennzeichnet, dass** der erste Strömungswiderstand ein den Fluidleitungspfad (5) lokal verengendes Kapillarelement (13) ist, das eine im Vergleich zu dem Förderdruck (p1) geringe Druckdifferenz (Δp) zwischen Förderdruck (p1) und Auslassdruck (p2) bewirkt, wobei vorzugsweise die Druckdifferenz (Δp) um einen Faktor 10 bis 20, bevorzugt 20 bis 100, besonders bevorzugt 100 bis 1.000, geringer ist als der Förderdruck (p1).

4. Infusionsanordnung (A) nach Anspruch 3, **dadurch gekennzeichnet, dass** das Kapillarelement (13) einen strömungswirksamen Querschnitt (Q) aufweist, der wenigstens 100 µm, bevorzugt 100 µm bis 150 µm, beträgt.

5. Infusionsanordnung (A) nach einem der vorhergehenden Ansprüche, **dadurch gekennzeichnet, dass** die Druckwaagenanordnung (15) ein mit dem Stellelement (16) wirkverbundenes Federelement (21) aufweist, das eine Federkraft (K) auf das Stellelement (16) bewirkt.

6. Infusionsanordnung (A) nach Anspruch 5, **dadurch gekennzeichnet, dass** eine Verstelleinrichtung (22) vorgesehen ist, mittels derer die Federkraft (K) des Federelements (21) manuell einstellbar ist.

7. Infusionsanordnung (A) nach einem der vorhergehenden Ansprüche, **dadurch gekennzeichnet, dass** das Stellelement in Form eines Druckkolbens (16a) oder in Form einer Druckmembran (16) ausgebildet ist.

8. Infusionsanordnung (A) nach Anspruch 7, **dadurch gekennzeichnet, dass** die Druckmembran (16) eine randseitig umlaufende Sickenanordnung (23) nach Art einer Lautsprechermembran aufweist.

## Claims

1. Infusion arrangement (A) for administering a medical fluid (F), having
- a medical elastomer pump (1) with an elastomer membrane (2) which forms a pump volume (3) for receiving and delivering the medical fluid (F), wherein the elastomer membrane (2), in a filling state of the pump volume (3) at least partially filled with the medical fluid (F), has an elastic expansion dependent on the filling state, and wherein the elastic expansion subjects the pump volume (3) to a delivery pressure (p1) that is dependent on expansion and thus variable over an infusion duration,
- a fluid conduit path (5) which is provided for discharging the medical fluid (F) from the pump volume (3) and which at one end is fluidically connected to an outlet (6) of the pump volume (3) and at the other end is fluidically connectable to a patient port (7), and
- a fluid control device (11, 11a) which is arranged in the fluid conduit path (5) and is designed to influence a volumetric flow (V) of the medical fluid (F) delivered through the fluid conduit path (5) by means of the elastomer pump (1),
- wherein the fluid control device (11, 11a) has a hydraulic flow-regulating valve (12, 12a), which is designed to regulate the volumetric flow (V) to a setpoint value and is provided with a throttle action (W, Wa) that is automatically variable at least in accordance with the delivery pressure (p1),
**characterized in that** the hydraulic flow-regulating valve is a 2-way flow-regulating valve (12, 12a) and has a first flow resistor (13), on which the delivery pressure (p1) acts at the inlet side and an outlet pressure (p2) acts at the outlet side, and a second flow resistor (14, 14a), which has the automatically variable throttle action (W, Wa) and is located downstream from the first flow resistor (13) in the delivery direction of the volumetric flow (V), and a pressure scale arrangement (15, 15a) with a hydraulically movable actuating element (16, 16a) which is subjected at one end to the delivery pressure (p1) and at the other end to the outlet pressure (p2) and by means of which the second flow resistor (14, 14a) is automatically variable in accordance with a movement of the actuating element (16, 16a) caused by differential pressure.

2. Infusion arrangement (A) according to claim 1, **characterized in that** the flow-regulating valve (12) has a constant control characteristic, wherein the throttle action (W) is automatically constantly variable, or **in that** the flow-regulating valve (12a) has a non-constant 2-point control characteristic, wherein the throttle action (Wa) is automatically alternately variable between a blocking and an enabling of the volumetric flow (V).

3. Infusion arrangement (A) according to claim 1 or 2, **characterized in that** the first flow resistor is a capillary element (13) which locally narrows the fluid conduit path (5) and which produces a pressure difference (Δp), which is small compared to the delivery pressure (p1), between delivery pressure (p1) and outlet pressure (p2), wherein the pressure difference (Δp) is preferably smaller than the delivery pressure (p1) by a factor of 10 to 20, preferably 20 to 100, particularly preferably 100 to 1000.

4. Infusion arrangement (A) according to claim 3, **characterized in that** the capillary element (13) has a flow-effective cross section (Q) which measures at least 100 µm, preferably 100 µm to 150 µm.

5. Infusion arrangement (A) according to any of the preceding claims, **characterized in that** the pressure scale arrangement (15) has a spring element (21), which is operatively connected to the actuating element (16) and subjects the actuating element (16) to a spring force (K).

6. Infusion arrangement (A) according to claim 5, **characterized in that** an adjustment device (22) is provided, by means of which the spring force (K) of the spring element (21) is manually adjustable.

7. Infusion arrangement (A) according to any of the preceding claims, **characterized in that** the actuating element is designed in the form of a pressure piston (16a) or in the form of a pressure membrane (16).

8. Infusion arrangement (A) according to claim 7, **characterized in that** the pressure membrane (16) has a bead arrangement (23) extending about the periphery in the manner of a loudspeaker diaphragm.

## Revendications

1. Agencement de perfusion (A) pour l'administration d'un fluide médical (F), présentant
- une pompe médicale élastomère (1) avec une membrane élastomère (2) qui réalise un volume de pompage (3) pour recevoir et transporter le fluide médical (F), la membrane élastomère (2) présentant, dans un état de remplissage du volume de pompage (3) au moins partiellement rempli du fluide médical (F), une dilatation élastique dépendant de l'état de remplissage, et la dilatation élastique provoquant une pression de transport (p1) dépendant de la dilatation et donc variable sur une durée de perfusion sur le volume de pompage (3),
- un trajet de conduite de fluide (5) prévu pour l'évacuation du fluide médical (F) du volume de pompage (3), qui est relié à une extrémité de manière à conduire le fluide à une sortie (6) du volume de pompage (3) et qui peut être relié à l'autre extrémité de manière à conduire le fluide à un accès de patient (7), et
- un dispositif de commande de fluide (11, 11a) agencé dans le trajet de conduite de fluide (5), qui est adapté pour influencer un débit volumique (V) du fluide médical (F) transporté à travers le trajet de conduite de fluide (5) au moyen de la pompe élastomère (1),
- le dispositif de commande de fluide (11, 11a) présentant une soupape de régulation de débit hydraulique (12, 12a) qui est adaptée pour réguler le débit volumique (V) à une valeur de consigne et qui est pourvue d'un effet d'étranglement (W, Wa) qui varie automatiquement au moins en fonction de la pression de transport (p1), **caractérisé en ce que** la soupape de régulation de débit hydraulique est une soupape de régulation de débit à 2 voies (12, 12a) et présente une première résistance à l'écoulement (13) à laquelle s'applique la pression de transport (p1) côté entrée et une pression de sortie (p2) côté sortie, et une deuxième résistance à l'écoulement (14, 14a), qui présente l'effet d'étranglement (W, Wa) qui varie automatiquement et qui est placée en aval de la première résistance à l'écoulement (13) dans la direction de transport du débit volumique (V), et un agencement de balance de pression (15, 15a) avec un élément de réglage (16, 16a) déplaçable hydrauliquement, qui est soumis à une extrémité à la pression de transport (p1) et à l'autre extrémité à la pression de sortie (p2) et au moyen duquel la deuxième résistance à l'écoulement (14, 14a) peut être modifiée automatiquement en fonction d'un déplacement de l'élément de réglage (16, 16a) dû à la pression différentielle.

2. Agencement de perfusion (A) selon la revendication 1, **caractérisé en ce que** la soupape de régulation de débit (12) présente une caractéristique de régulation continue, l'effet d'étranglement (W) étant variable en continu de manière automatique, ou **en ce que** la vanne de régulation de débit (12a) présente une caractéristique de régulation à 2 points non continue, l'effet d'étranglement (Wa) étant variable de manière automatique alternativement entre un blocage et une libération du débit volumique (V).

3. Agencement de perfusion (A) selon la revendication 1 ou 2, **caractérisé en ce que** la première résistance à l'écoulement est un élément capillaire (13) rétrécissant localement le trajet de conduite de fluide (5), qui provoque une faible différence de pression (Δp) entre la pression de transport (p1) et la pression de sortie (p2) en comparaison de la pression de refoulement (p1), la différence de pression (Δp) étant de préférence inférieure à la pression de transport (p1) d'un facteur 10 à 20, de préférence 20 à 100, de manière particulièrement préférée 100 à 1000.

4. Agencement de perfusion (A) selon la revendication 3, **caractérisé en ce que** l'élément capillaire (13) présente une section transversale (Q) efficace pour l'écoulement, qui est d'au moins 100 µm, de préférence de 100 µm à 150 µm.

5. Agencement de perfusion (A) selon l'une quelconque des revendications précédentes, **caractérisé en ce que** l'agencement de balance de pression (15) présente un élément de ressort (21) en liaison active avec l'élément de réglage (16), qui exerce une force de ressort (K) sur l'élément de réglage (16).

6. Agencement de perfusion (A) selon la revendication 5, **caractérisé en ce qu'**il est prévu un dispositif de réglage (22) au moyen duquel la force de ressort (K) de l'élément de ressort (21) peut être réglée manuellement.

7. Agencement de perfusion (A) selon l'une quelconque des revendications précédentes, **caractérisé en ce que** l'élément de réglage est réalisé sous la forme d'un piston de pression (16a) ou sous la forme d'une membrane de pression (16).

8. Agencement de perfusion (A) selon la revendication 7, **caractérisé en ce que** la membrane de pression (16) présente un agencement de moulure (23) périphérique sur le bord, à la manière d'une membrane de haut-parleur.
